# EUROPEAN PATENT APPLICATION

(11) **EP 4 542 567 A1**
(43) Date of publication of application: **23.04.2025**
(21) Application number: 23203706.9
(22) Date of filing: 16.10.2023
(51) Int. Cl.: G16H 40/63, A61B 5/00, A61B 5/0205, A61B 5/0245

(54) **DEVICE AND METHOD FOR CONTROLLING THE ISSUANCE OF MEDICAL ALARM SIGNALS**

(71) Applicant: Koninklijke Philips N.V., 5656 AG Eindhoven (NL)
(72) Inventor: WALDEN, Andreas, Eindhoven (NL)
(74) Representative: Philips Intellectual Property & Standards

(57) **Abstract**

The present invention relates to device and method for controlling the issuance of medical alarm signals. The device comprises an input (21) configured to obtain a physiological signal of a subject, a processing unit (22) configured to generate a control signal and an output (23) configured to output the control signal to an alarm unit (30). The processing unit is configured to determine if and by which amount a vital sign value derived from or represented by the obtained physiological signal exceeds a vital sign threshold for said type of vital sign; set an alarm interval between two subsequent issuances of an alarm signal to be issued if the vital sign value exceeds the vital sign threshold based on the determined amount of exceedance of the alarm threshold, wherein the alarm interval is set lower the higher the amount of exceedance is; and generate a control signal for controlling the alarm unit (30) to issue medical alarm signals at the set alarm intervals.

## Description

### FIELD OF THE INVENTION

The present invention relates to a device and method for controlling the issuance of medical alarm signals. The present invention relates further to a system for issuing medical alarm signals.

### BACKGROUND OF THE INVENTION

Patient monitoring alarms in hospitals, particularly in intensive care units (ICUs), can significantly impact patient rest and sleep, making it essential for manufacturers of patient monitoring equipment to prioritize minimizing disturbance to patients. The alarms, typically consisting of visual and audible signals, are designed to grab caregivers' attention in multiple ways. However, the audible signal is often the main cause of alarm fatigue, patient stress, and sleep disruption.

Alarm fatigue is a phenomenon that occurs when healthcare providers are exposed to an excessive number of alarms and become desensitized to them, leading to delayed or missed responses to alarms. The problem of alarm fatigue is particularly prevalent in hospital settings, specifically in ICUs, where patients are closely monitored by medical equipment that generates a high volume of alarms. This constant exposure to alarms can lead to providers ignoring alarms or not responding to them promptly, potentially resulting in missed or delayed treatment for patients and subsequent complications. Additionally, alarm fatigue can also negatively impact the work environment for healthcare providers, leading to increased stress and burnout. Overall, alarm fatigue is a significant problem in hospital settings that can have serious consequences for both patients and healthcare providers.

### SUMMARY OF THE INVENTION

It is an object of the present invention to reduce noise caused by audible alarms and to reduce alarm fatigue of caregivers. It is a further object of the present invention to improve care and comfort of patients (or, more generally, subjects) and to provide an effective and safe alarm system.

In a first aspect of the present invention a device for controlling the issuance of medical alarm signals is presented, the device comprising:
an input configured to obtain a physiological signal of a subject;
a processing unit configured to
   - determine if and by which amount a vital sign value derived from or represented by the obtained physiological signal exceeds a vital sign threshold for said type of vital sign;
   - set an alarm interval between two subsequent issuances of an alarm signal to be issued if the vital sign value exceeds the vital sign threshold based on the determined amount of exceedance of the alarm threshold, wherein the alarm interval is set lower the higher the amount of exceedance is; and
   - generate a control signal for controlling an alarm unit to issue medical alarm signals at the set alarm intervals; and
an output configured to output the control signal to the alarm unit.

In a further aspect of the present invention a system for issuing medical alarm signals is presented, the system comprising:
a device configured to control the issuance of medical alarm signals as disclosed herein; and
an alarm unit configured to issue medical alarm signals at alarm intervals set by the device.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to adaptively set the time intervals (herein called alarm intervals, i.e., the time period in between two subsequent issuances of an alarm signal) based on the health condition of the subject. Instead of repeating alarm signals (e.g. alarm sounds or alarm light signals) at equidistant alarm intervals, the alarm intervals are variable and set based on the subject's current vital sign value (e.g. heart rate (HR) value and/or respiration rate (RR) value). More particularly, the subject's current vital sign value is compared to a corresponding vital sign threshold (e.g. a HR threshold or a RR threshold, also called alarm limits for HR or RR). The more the vital sign value exceeds the vital sign threshold (i.e., the more critical the patient's health status gets), the lower is the alarm interval set, i.e., the alarm signal will be issued more often (with shorter pauses in between two subsequent issuance of the alarm signal). In an embodiment, vice versa, if the vital sign value exceeds the vital sign threshold by a smaller amount, the alarm interval is increased, i.e., the alarm signal will be issued less often, i.e., the alarm interval is set higher the lower the amount of exceedance is.

In this way, for instance for a patient with a slightly elevated HR (compared to the HR threshold used as alarm limit) the alarm signal will be repeatedly issued with larger pauses in between compared to a patient with a much higher, almost critical HR. In this way, the presented approach considers the nuances of a patient's health condition and avoids, or at least reduces, unnecessary noise and alarm fatigue. On the other hand, it helps to improve caregiver's awareness of the patient's current health condition and to recognize a really critical health condition more quickly and easily. Still further, an effective and safe alarm system can be maintained by announcing alarms according to their urgency.

According to a preferred embodiment the processing unit is configured to determine if and by which amount the vital sign value exceeds an alarm threshold for said type of vital sign by determining if and by which amount the vital sign value is below a lower vital sign threshold and/or by determining if and by which amount the vital sign value is above an upper vital sign threshold. Thus, depending on the kind of vital sign, an upper and/or a lower vital sign threshold may be used for the comparison with the current vital sign signal value. For instance, in case of using HR as vital sign, a lower HR threshold, below which the HR gets increasingly critical, and an upper HR threshold, above which the HR gets increasingly critical as well, will be used. The different between the current HR and the respective HR threshold (i.e. the upper HR threshold in case of a high HR and the lower HR threshold in case of a low HR) is used then to set the alarm interval.

According to another embodiment the processing unit is configured to set the alarm interval not lower than a minimum alarm interval. This avoids e.g. too much noise in case of audible alarm sounds as alarm signals.

The processing unit may be configured to repeatedly or continuously update the setting of the alarm interval based on repeated or continuous measurements of the physiological signal. This improves the accuracy of the alarm signaling since a subject's health condition may quickly change.

In an embodiment the processing unit is configured to linearly or exponentially reduce the alarm interval in response to a linear increase of the amount of exceedance. The manufacturer or user may thus determine how fast the alarm interval may be reduced in case of an increasing amount of exceedance. This may increase the caregiver's awareness of an increasingly critical health condition of the subject.

The processing unit may preferably be configured to
- determine if and by which amount two or more vital sign values of different types of vital signs derived from or represented by one or more obtained physiological signal exceed a respective vital sign threshold for the respective type of vital sign;
- determine, per determined amount of exceedance of the two or more amounts of exceedance, a provisional alarm interval based on the respective amount of exceedance; and
- set the alarm interval to the minimum provisional alarm interval of the determined provisional alarm intervals or to an average of the determined provisional alarm intervals.
In this way, the subject's health condition may be more precisely taken into account.

The one or more vital signs derived from or represented by one or more physiological signals may be one or more of heart rate, respiration rate, oxygen saturation, body temperature, blood pressure, and end tidal carbon dioxide. It is noted that the invention can not only be used with one or more of these vital signs, but with one or more other vital signs as well, which may be used for checking / deciding if there is a critical situation or alarm situation for the subject.

According to an aspect of the present invention, the system for issuing medical alarm signals comprises a device as disclosed herein, and an alarm unit configured to issue medical alarm signals at alarm intervals set by the device. Hereby, the alarm unit may be configured to issue audible and/or visual alarm signals, e.g. in the form of short tones and/or short light blinking. The alarm unit may thus comprise a loudspeaker, a display, a light source (e.g. LED), etc.

The system may be implemented in different ways. In a preferred implementation the system may be implemented in or as a portable (wearable, mobile) or stationary patient monitor or a central station configured to obtain physiological signals and/or alarm signals from multiple subjects. The central station may be a central computer or workstation or other device, where alarms and vitals from multiple patients are centralized, e.g. in an ICU unit. In an embodiment, only the alarm of the highest priority may then be announced. In this way, the user of the central station, e.g. a nurse or caregiver, will get more nuanced feedback (e.g. audible feedback of the alarm with the highest priority) for the entire patient population that is monitored at this central station. The situation awareness at this central location, e.g. at the ICU, can thus be improved.

The system may further comprise one or more sensors configured to acquire one or more physiological signals of the subject. The one or more sensors may include one or more of a heart rate sensor, pulse oximeter, respiration rate sensor, oxygen saturation sensor, body temperature sensor, blood pressure (BP) measurement device, end tidal carbon dioxide sensor. The physiological signal may e.g. be a photoplethysmography (PPG) signal, a ballistocardiogram (BCG) signal, a movement signal reflecting e.g. chest movements caused by respiration, etc.

### BRIEF DESCRIPTION OF THE DRAWINGS

These and other aspects of the invention will be apparent from and elucidated with reference to the embodiment(s) described hereinafter. In the following drawings
Fig. 1 shows a diagram of a static alarm interval curve.
Fig. 2 shows a diagram of a variable alarm interval curve.
Fig. 3 shows a diagram of the comparison of a static alarm interval curve and a variable alarm interval curve.
Fig. 4 shows a schematic diagram of an embodiment of a system according to the present invention.
Fig. 5 shows a schematic diagram of an embodiment of a device according to the present invention.
Fig. 6 shows a schematic diagram of an embodiment of a method according to the present invention.

### DETAILED DESCRIPTION OF EMBODIMENTS

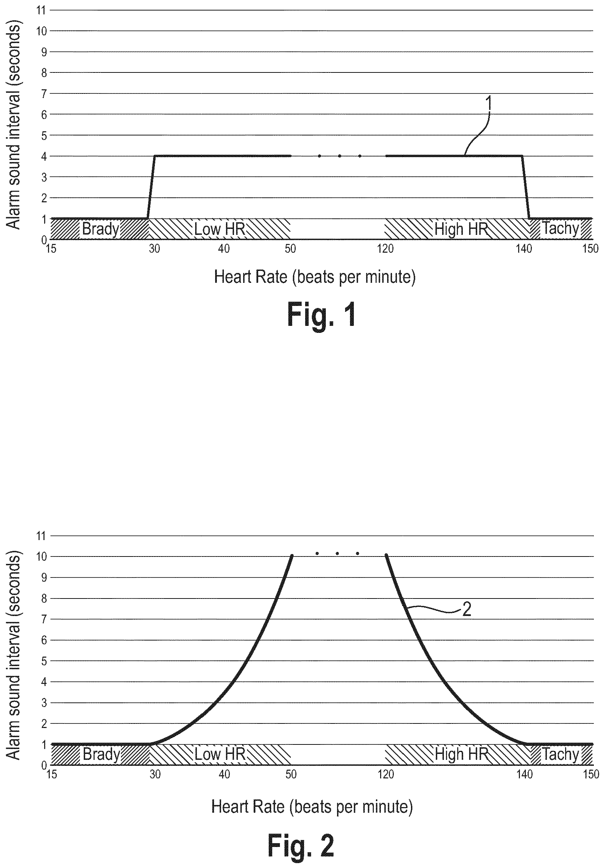

Nowadays, alarm sounds are repeated at equidistant intervals, which may vary depending on the alarm's priority level (low, medium, high). A low-priority alarm sound may be repeated every six seconds in a low priority range (e.g. normal HR with beats per minute (BPM) in the range of around 50-120), a medium-priority every four seconds in medium priority ranges (e.g. low HR with BPM in the range of around 30-50 or high HR with BPM in the range of around 120-140), whereas a high-priority alarm may be repeated each second in high priority ranges (e.g. very low HR with BPM below around 30 also called brachycardia region ("brady") or very high HR with BPM above around 140 also called tachycardia region ("tachy")). Thus, a medium-priority alarm, for example, might be announced at an elevated HR in the range of 120 to 140 (or even higher) BPM, i.e., for a HR of 120 and 140 the same alarm signal with the same alarm interval is issued. A diagram showing the use of such a static alarm interval curve 1 is shown in Fig. 1.

The unnuanced design of the alarm might not reflect the criticality of the patient on both ends of the spectrum of that range of the heart rate. There might be too much noise when the patient's heart rate is closer to the lower spectrum of the range and not gaining enough attention at the higher end. Furthermore, this approach does not consider the nuances of a patient's condition, leading to unnecessary noise and alarm fatigue decreased situation awareness.

To increase situational awareness and reduce unnecessary situational noise, according to embodiments of the present invention the interval pause, e.g. for the respective alarm priority, is computed based on the interval range of the corresponding vital sign. The transition between the alarm priorities regarding their interval pause (i.e., alarm interval) is preferably made seamless to ensure that nuances in the vital signs are reflected, increasing situational awareness.

Fig. 2 shows a diagram of the use of a variable alarm interval curve 2. It shows how the interval pause curve 2 starts at a more relaxed value of 10 seconds for HR values in the "normal" (healthy) range, which indicates less urgency which is in line with the set alarm limit. The interval pause declines in the range of BPM and reaches a value of four seconds in the middle of the low HR range (around 40 BPM) and in the middle of the high HR range (around 130 BPM), which reflects the urgency of a lower HR and higher HR, respectively. Finally, the interval pause reaches a value of one second just before transitioning into a high-priority alarm (i.e., the brady region or tachy region, respectively).

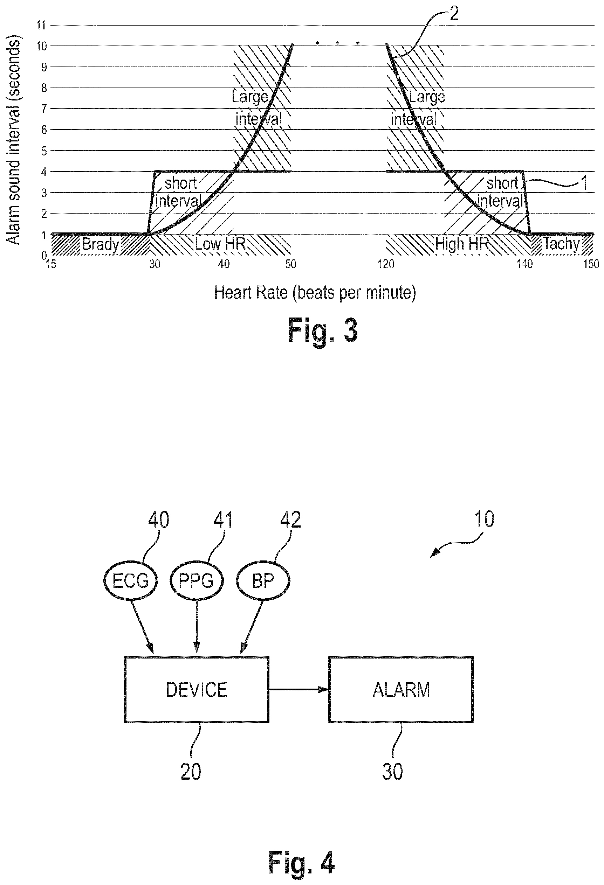

The comparison of the static interval time and the interval curve shown in the diagram depicted in Fig. 3 shows how the interval curve indicates less urgency through larger interval pause times closer to the start of the medium-priority alarm limits (e.g. around 50 and 120 BPM, respectively) and more urgency through shorter interval pause times closer to the high-priority alarm limits (e.g. around 30 and 140 BPM, respectively).

The decrease of the alarm limit with increasing amount of exceedance of the alarm threshold (and vice versa) can be made steadily / continuously, as shown in Figs. 2 and 3. In other embodiments, the decrease or increase, respectively, can be made in multiple small steps.

According to preferred embodiments of the present invention, the relative vital sign value may be compared to its low, medium, and high priority levels, and this information may be applied to the interval pause of the audible signal of the alarm. This approach allows for much wider interval pauses in areas of transitions between priority levels, resulting in less noise for both patients and caregivers. This approach does not only help to improve the patient's recovery but also helps to avoid alarm fatigue of the caregivers and leads to better patient care.

In Figs. 1-3 HR is used as example of a vital sign to demonstrate the use case. In general, multiple vital signs can be acquired simultaneously, and each one can trigger multiple alarms. The most common alarms are so-called medium priority alarms, indicating that an alarm limit (High/Low) is exceeded. This alarm may trigger a specific tone every two seconds without giving further context of how much the limit has been exceeded. In an embodiment the different alarm sources (e.g., HR high, SpO2 low, RR high, etc. ) may be mixed in a way that always the most critical alarm interval is used for the issuance of the alarm signal. In case of using an alarm sound as alarm signal, this is still beneficial regarding alarm noise reduction and provides improved situation awareness through the dynamic nature of this new behavior.

In a hospital room, multiple alarms may be issued by different patient monitors. Adapting the alarm intervals based on the condition of the respective patients may let the caregiver detect which patient monitor (and therefore patient) needs more attention.

Fig. 4 shows a schematic diagram of an embodiment of a system 10 for issuing medical alarm signals according to the present invention. The system 10 comprises a device 20 that is configured to control the issuance of medical alarm signals and an alarm unit 30 that is configured to issue medical alarm signals at alarm intervals set by the device 20. The device 20 may thus generate and provide a control signal to the alarm unit 30, which then issues the medical alarm signals according to the control signal.

The system 10 may be a (portable or stationary) patient monitor that monitors multiple vital signs of a patient, e.g. HR, RR, SpO2, BP, etc. and that may generate a medical health score (e.g. an early warning score, EWS). For this purpose the system obtains one or more physiological signals of the subject that have been acquired by one or more sensors 40, 41, 42. For instance, an ECG sensor 40 may acquire ECG signals allowing to derive HR and other HR related vital signs. A pulse oximeter 41 may acquire PPG signals allowing to derive HR and SpO2. A blood pressure sensor 42 may acquire BP signals. Further sensors may be provided to acquire further physiological signals and/or further vital sign signals, such as , body temperature and end tidal carbon dioxide. Further and/or other sensors may be provided. In other embodiments, the system 10 may be implemented in a computer, tablet, workstation, smartphone or other device that is configured to carry out the functions of the system 10.

The device 20 obtains the one or more physiological signals acquired by the one or more sensors 40-42 and derives vital signs from them, or directly obtains one or more vital signs acquired by the one or more sensors 40-42. The device 20 may be implemented by respective units or circuitry, e.g. a processor, processing circuitry, a computer, dedicated hardware, etc., that carries out the functions of the device. Alternatively, a common unit or circuitry, e.g. a common processor or computer, may implement the various functions of the device, or separate units or elements may be used that together represent the circuitry. In an exemplary implementation, a programmed processor may represent the device 20, which may execute a computer program that may be stored in a memory that is accessed by the processor.

The alarm unit 30 is configured to issue audible and/or visual alarm signals. The alarm unit may e.g. comprise a loudspeaker and/or a display, which may be integrated into a patient monitor.

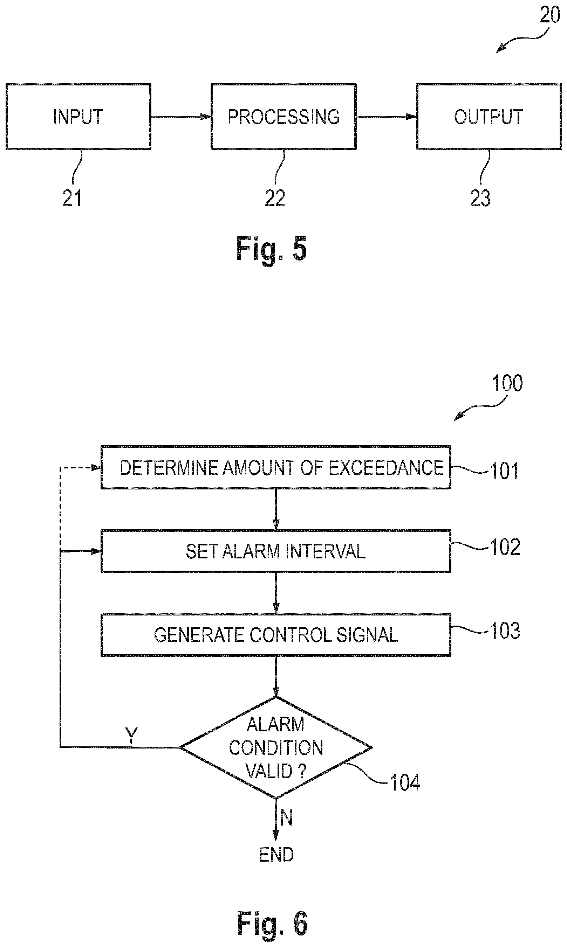

Fig. 5 shows a schematic diagram of an embodiment of a device 20 according to the present invention. The device 20 comprises an input 21 that is configured to obtain a physiological signal (or multiple physiological signals) of a subject. The input 21 may be directly coupled or connected to the one or more sensors 40-42 or may obtain (i.e. retrieve or receive) these signal(s) from a storage, buffer, network, or bus, etc. The input 21 may thus e.g. be a (wired or wireless) communication interface or data interface, such as a Bluetooth interface, Wi-Fi interface, LAN interface, HDMI interface, direct cable connection, or any other suitable interface allowing signal transfer to the device 20.

The device 20 further comprises a processing unit 22 configured to carry out various steps and generate a control signal. The processing unit 22 may be any kind of means configured to process the obtained physiological signal(s) and generate the control signal. It may be implemented in software and/or hardware, e.g. as a programmed processor or computer or app on a user device such as a smartphone, smartwatch, tablet, laptop, PC, workstation, etc.

The device 20 further comprises an output 23 that is configured to output the control signal to the alarm unit 30. The output 23 may generally be any interface that provides the control signal, e.g. transmits it to the alarm unit 30 or provides it for retrieval by the alarm unit 30. It may thus generally be any (wired or wireless) communication or data interface.

In another embodiment the system 10 may be implemented in or as a central station at which multiple patients are monitored, e.g. in a central station at the ICU or a department of a hospital. The central station may either receive (or retrieve) alarm signals from multiple patients, e.g. from multiple patient monitors of multiple patients, and/or it may receive (or retrieve) physiological signals from multiple patients, e.g. from multiple patient monitors (or even directly from sensors) of multiple patients. In the latter case, the central station may itself process the physiological signals and determine if there is an alarm situation and may then itself generate control signals for the alarm unit, which may be part of the central station as well so that itself outputs the alarm according to the control signal and as herein described. This will increase the awareness of the users of the central station which may be essential if a larger number of patients are centrally monitored.

Fig. 6 shows a schematic diagram of an embodiment of a method 100 according to the present invention. The steps of the method 100 may be carried out by the device 20, wherein the main steps of the method 100 are carried out by the processing unit 22. The method may e.g. be implemented as computer program running on a computer or processor.

In a first step 101, it is determined if and by which amount a vital sign value derived from or represented by the obtained physiological signal exceeds a vital sign threshold for said type of vital sign. For instance, for HR a HR threshold may be used, and for SpO2 an SpO2 threshold may be used.

In a second step 102, an alarm interval between two subsequent issuances of an alarm signal to be issued is set if the vital sign value exceeds the vital sign threshold based on the determined amount of exceedance of the alarm threshold, wherein the alarm interval is set lower the higher the amount of exceedance is.

In a third step 103, a control signal for controlling the alarm unit 30 to issue medical alarm signals at the set alarm intervals is generated.

In a subsequent optional step 104 it may be checked if the alarm condition is still valid. If it is valid the method may continue with step 101 or step 102; otherwise, it may end the method (and e.g. increase the alarm interval to a "normal" value).

In an embodiment, in step 101, a lower threshold and an upper threshold may be used to determine if the patient gets into a critical condition. The amount (i.e. level or seriousness) of the criticality may be determined based on the difference between the current vital sign signal and the respective threshold. For instance, referring to Fig. 2, an upper HR threshold may be 120 BPM and a lower HR threshold may be 50 BPM. If the current HR is 130 BPM, it deviates by 10 BPM from the upper HR threshold. If the current HR is 40 BPM, it deviates by 10 BPM from the lower HR threshold, i.e. the amount of deviation from the respective threshold is the same. In an embodiment this may lead to the same alarm interval. In other embodiments, different alarm intervals may be set depending on whether an upper or lower threshold has been exceeded.

In an embodiment, in step 102 the alarm interval may not be set lower than a minimum alarm interval. For instance, with reference to Fig. 2, the alarm interval may not be lower than 1 second.

The computation and setting described above may be repeatedly or continuously carried out to update the setting of the alarm interval based on repeated or continuous measurements of the physiological signal. This contributes to ensuring awareness of the caregiver if the patient's condition has suddenly worsened so that the alarm interval should be reduced and avoids alarm fatigue if a critical situation is no longer given, and the alarm interval can thus be increased again.

As shown in Fig. 2, the alarm interval may be exponentially reduced in response to a linear increase of the amount of exceedance. However, other behaviors of the increase may be implemented as well, e.g. a linear increase with a desired slope.

A practical implementation may use the following components:
a) a list of all vital parameter alarms and their respective alarm ranges;
b) a function in the patient monitor that calculates the modifiers of a formula initially for each vital parameter limit and in the event of any alarm limit change;
c) a function in the patient monitor that calculates the corresponding interval pause time for each parameter alarm according to the function and modifiers of component b);
d) a function that compares the interval pause values and returns the smallest interval pause; and
e) a function that announces the audible alarm signal according to the resulting interval pause.

An exemplary formula used by component b) may be: *f*(*x*) = *b*^{(*c*∗*x*+*d*)}, with b being the base with 0 < b < 1 for high alarm limits and b > 1 for low alarm limits, c being an exponent, x being an independent variable and d being a translation. If b > 1, the function represents exponential growth. As x increases, the corresponding y values increase rapidly.

If 0 < b < 1, the function represents exponential decay. As x increases, the corresponding y values decrease rapidly. The base essentially determines the shape and direction of the exponential curve in the function.

In an exemplary example using HR as vital sign, the following exemplary numbers may be used for the parameters in this exemplary formula:
i) Low Alarm Limit Range (HR between 30 and 50 BPM): b=1.4; c=0.1000015828; x = 0 to 20 (difference of heat beat from alarm limit 30 to 50 with an offset of -30); d=-0.4.
ii) High Alarm Limit Range (HR between 120 and 140 BPM): b=0.891; c=1; x=120 to 140; d=-140.
iii) Below 30 BPM and above 140 BPM, the interval pause is set to 1 sec, as it uses the high priority alarm sound. The range of 51 to 119 BPM will not generate any alarms.

By use of the present invention, e.g. in patient monitoring, noise and alarm fatigue can be reduced. A higher alarm interval between the alarm sounds corresponds to noise reduction. Further, recognizing priority/criticality of a patient condition is improved. Generally, in patient monitoring, an alarm is always to be taken seriously, but relative criticality can be improved by the present invention, which helps the caregiver in the prioritization in case of several alarms given by different patient monitors monitoring different patients. Making a decision which patient (out of several) should be attended first can be supported by the present invention. By announcing alarms according to their urgency an effective and safe alarm system can be maintained.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (20) for controlling the issuance of medical alarm signals, the device comprising:
an input (21) configured to obtain a physiological signal of a subject;
a processing unit (22) configured to
- determine if and by which amount a vital sign value derived from or represented by the obtained physiological signal exceeds a vital sign threshold for said type of vital sign;
- set an alarm interval between two subsequent issuances of an alarm signal to be issued if the vital sign value exceeds the vital sign threshold based on the determined amount of exceedance of the alarm threshold, wherein the alarm interval is set lower the higher the amount of exceedance is; and
- generate a control signal for controlling an alarm unit (30) to issue medical alarm signals at the set alarm intervals; and
an output (23) configured to output the control signal to the alarm unit.
wherein the processing unit (22) is configured to set the alarm interval higher the lower the amount of exceedance is.

2. Device according to claim 1,
wherein the processing unit (22) is configured to determine if and by which amount the vital sign value exceeds an alarm threshold for said type of vital sign by determining if and by which amount the vital sign value is below a lower vital sign threshold and/or by determining if and by which amount the vital sign value is above an upper vital sign threshold.

3. Device according to any preceding claim,
wherein the processing unit (22) is configured to set the alarm interval not lower than a minimum alarm interval.

4. Device according to any preceding claim,
wherein the processing unit (22) is configured to repeatedly or continuously update the setting of the alarm interval based on repeated or continuous measurements of the physiological signal.

5. Device according to any preceding claim,
wherein the processing unit (22) is configured to linearly or exponentially reduce the alarm interval in response to a linear increase of the amount of exceedance.

6. Device according to any preceding claim,
wherein the processing unit (22) is configured to
- determine if and by which amount two or more vital sign values of different types of vital signs derived from or represented by one or more obtained physiological signal exceed a respective vital sign threshold for the respective type of vital sign;
- determine, per determined amount of exceedance of the two or more amounts of exceedance, a provisional alarm interval based on the respective amount of exceedance; and
- set the alarm interval to the minimum provisional alarm interval of the determined provisional alarm intervals or to an average of the determined provisional alarm intervals.

7. Device according to any preceding claim,
wherein one or more vital signs derived from or represented by one or more physiological signals is one or more of heart rate, respiration rate, oxygen saturation, body temperature, blood pressure, and end tidal carbon dioxide.

8. Device according to any preceding claim,
wherein the processing unit (22) is configured to set the alarm interval higher the lower the amount of exceedance is.

9. System (10) for issuing medical alarm signals, the system comprising:
a device (20) configured to control the issuance of medical alarm signals according to any preceding claim; and
an alarm unit (30) configured to issue medical alarm signals at alarm intervals set by the device.

10. System according to claim 9,
wherein the alarm unit (30) is configured to issue audible and/or visual alarm signals.

11. System according to claim 9 or 10,
wherein the system is a portable or stationary patient monitor, or a central station configured to obtain physiological signals and/or alarm signals from multiple subjects.

12. System according to any one of claims 9 to 11,
further comprising one or more sensors (40, 41, 42) configured to acquire one or more physiological signals of the subject.

13. Method for controlling the issuance of medical alarm signals, the method comprising:
- obtaining a physiological signal of a subject;
- determining if and by which amount a vital sign value derived from or represented by the obtained physiological signal exceeds a vital sign threshold for said type of vital sign;
- setting an alarm interval between two subsequent issuances of an alarm signal to be issued if the vital sign value exceeds the vital sign threshold based on the determined amount of exceedance of the alarm threshold, wherein the alarm interval is set lower the higher the amount of exceedance is;
- generating a control signal for controlling an alarm unit (30) to issue medical alarm signals at the set alarm intervals; and
- outputting the control signal to the alarm unit.

14. Computer program comprising program code means for causing a computer to carry out the steps of the method as claimed in claim 13 when said computer program is carried out on the computer.
